(19) Europäisches Patentamt / European Patent Office / Office européen des brevets

(11) **EP 3 834 887 A1**

(12) **EUROPEAN PATENT APPLICATION**

(43) Date of publication:
**16.06.2021 Bulletin 2021/24**

(21) Application number: **19215655.2**

(22) Date of filing: **12.12.2019**

(51) Int Cl.:
*A61P 1/02* (2006.01)     *A61K 9/00* (2006.01)
*A61K 9/06* (2006.01)     *A61K 47/10* (2017.01)
*A61K 47/34* (2017.01)     *A61K 47/36* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(71) Applicant: **Koninklijke Philips N.V.**
**5656 AG Eindhoven (NL)**

(72) Inventors:
• **GOTTENBOS, Bart**
  **5656 AE Eindhoven (NL)**
• **MAROT, Melanie**
  **5656 AE Eindhoven (NL)**

(74) Representative: **Philips Intellectual Property & Standards**
**High Tech Campus 5**
**5656 AE Eindhoven (NL)**

(54) **TOPICAL ORAL COMPOSITION**

(57)     Provided is an aqueous topical oral composition. The composition is suitable for applying within the oral cavity, e.g. to teeth and/or gums. The composition comprises a poloxamer for increasing the complex viscosity of the composition at a physiological temperature, e.g. ≥33°C, such as about 37°C, which is higher relative to room temperature, e.g. ≤25°C. The poloxamer thus thickens the composition upon application within the oral cavity, thereby improving its substantivity therein. The poloxamer has a degree of polymerization corresponding to the total number of monomer units of the poloxamer. The composition further includes an additive in the form of a polysaccharide. The additive is soluble in the aqueous composition at room temperature. It follows that the additive is also soluble in the composition at the physiological temperature. The polysaccharide has a degree of polymerization corresponding to the total number of monomer units of the polysaccharide. The degree of polymerization of the polysaccharide is higher than the degree of polymerization of the poloxamer. Further provided is a kit for preparing the composition, an apparatus for applying the composition, and methods comprising application of the composition within the oral cavity.

FIG. 15

**Description**

FIELD OF THE INVENTION

This invention relates to a topical oral composition, an apparatus comprising the composition and an applicator, a kit for preparing the composition, and methods and uses of the composition, particularly involving its application within the oral cavity.

BACKGROUND OF THE INVENTION

Many active chemical agents such as hydrogen peroxide, fluoride and antimicrobial compounds are used in oral care. Their efficacy depends not only on their concentration, but also on the time they can react on the teeth or gums. The latter corresponds to the contact time or residence time of the active agent within the oral cavity. Compositions for application within the oral cavity need to be fluidic or semifluidic for practical use in combination with applicators, such as toothbrushes or delivery syringes, or for use as mouthwashes. This requirement typically limits the residence time in the oral cavity because salivary flow can wash away the composition relatively quickly.

Various approaches have been employed in order to provide sustained release of active agents from oral compositions. One such approach involves using solutions which form gels in situ; such gels having increased substantivity (adherence to the surface of the teeth or gums) in the oral cavity. A disadvantage of in situ gelling compositions is that application tends to be complicated by the steps required to generate the gel prior to application. For example, combining two components which are initially supplied separately from each other is first required before the in situ formed gel can be applied. In another example, an electrochemically driven pH increase is first required in order to form a crosslinked gel.

Chitosan-containing gels, for instance, have a relatively high substantivity in the oral cavity, but have, as explained above, the disadvantage that they tend to require mixing with a second component in order for in situ crosslinking to take place to form gels. Additionally, the delivery and crosslinking needs a specific complex methodology to make sure the chitosan gel adheres properly to the teeth.

Thermoresponsive compositions, which are liquid upon application when at room temperature but form gels in the oral cavity due to the elevated (physiological) temperature, offer a more easy-to-use class of in situ gelling system. Such compositions may be provided as a single solution, without any requirement to mix with another component, and are thus simple to apply.

The thermoresponsive behavior may be provided by including a poloxamer in the composition. Poloxamers are nonionic triblock copolymers composed of a central hydrophobic chain of polyoxypropylene (poly(propylene oxide)) flanked by two hydrophilic chains of polyoxyethylene (poly(ethylene oxide)). Poloxamers are also known by the trade names Synperonics, Pluronics, and Kolliphor. These block co-polymers form loose micelles at lower temperatures, but organize in long strings at elevated temperatures, thereby increasing the viscosity of the composition (Hamley, I. W., The physics of block copolymers, Oxford University Press; New York, USA, pp. 1-20, 1998).

In the Philips Zoom! whitening products, for example, Poloxamer 407 (Pluronic F127) is included in the composition at a concentration of 25 wt.%. The whitening composition can be easily pushed out of a syringe at room temperature, but on the teeth the elevated temperature causes the viscosity of the composition to increase, thereby assisting to hinder flow of the composition away from the teeth.

It remains a challenge to improve the properties of such thermoresponsive oral compositions.

SUMMARY OF THE INVENTION

The invention is defined by the claims.

According to an aspect there is provided an aqueous topical oral composition comprising: a poloxamer for increasing the complex viscosity of the composition at a physiological temperature relative to room temperature, the poloxamer having a first degree of polymerization corresponding to the total number of monomer units of the poloxamer; and an additive in the form of a polysaccharide dissolved in the composition, the polysaccharide having a second degree of polymerization corresponding to the total number of monomer units of the polysaccharide which is higher than the first degree of polymerization of the poloxamer.

Conventional poloxamer-containing oral compositions have been found to have disadvantageously low resistance to dissolution in saliva. This means that saliva flowing around the applied composition (in the form of a gel inside the oral cavity) causes the composition to be relatively quickly, e.g. within minutes, washed away. This observation led to such poloxamer-containing oral compositions being initially discarded for applications requiring longer term sustained release of active agents in the oral cavity.

The present invention is based on the realization that the resistance of a poloxamer-containing aqueous topical oral composition to dissolution in saliva may be increased by including an additive in the form of a polysaccharide in the composition. In particular, by the polysaccharide having a (second) degree of polymerization corresponding to the total number of monomer units, i.e. ring moieties, of the polysaccharide which is higher than a (first) degree of polymerization of the poloxamer, the additive may decrease the solubility of the in situ gelled composition applied within the oral cavity. Without wishing to be bound by any particular theory, gelation of the poloxamer at the physiological temperature may coincide with the larger polysaccharide molecules providing entanglements within the gel, which entanglements restrict motion of the poloxamer molecules within the gel. The entanglements thereby reduce the aqueous solubility of the gelled composition relative to a composition which does not include such a polysaccharide additive.

This means that the gelled composition is less susceptible to being washed away by saliva, and therefore remains applied to a surface, e.g. on a tooth, between teeth or on gums, etc., for a longer period of time relative to a composition which does not include such an additive. The composition is correspondingly particularly useful for, for example, sustained release of an active agent included in the composition, e.g. over a number of hours, such as overnight. In other examples in which the composition does not include an active ingredient, the gelled composition may provide a barrier for covering or protecting an area within the oral cavity over such a sustained period.

Moreover, the polysaccharide may be non-toxic, thereby rending the additive particularly suitable for increasing the dissolution resistance of the gelled topical oral composition.

The composition is aqueous, such that the gelled composition at the physiological temperature may be regarded as a hydrogel. Water may thus constitute the main or base component of the composition. The composition may comprise, for example, 70 to 90 wt.%, such as 75 to 95 wt.%, e.g. 78 to 84 wt.% of water.

The additive is dissolved in the aqueous composition. The additive may thus be soluble in the composition at room temperature, and may have a relatively low concentration in the composition of 0.5 to 5 wt.%, such as 0.8 to 2.5 wt.%, e.g. 1 to 2 wt.%. It has been found that keeping the additive concentration within these ranges advantageously provides the above-described dissolution resistivity effect at the physiological temperature but assists to ensure a low enough viscosity at room temperature such that the composition is straightforward to apply, e.g. inject, within the oral cavity.

The poloxamer may have a triblock copolymer structure having the general formula: $[CH_2CH_2O]_x[CH(CH_3)CH_2O]_y[CH_2CH_2O]_z$, and the first degree of polymerization may be the sum of x, y and z.

The first degree of polymerization of the poloxamer may be in the range of 50 to 300. A poloxamer having a (first) degree of polymerization within this range may assist with attaining a composition having the desired viscosity at room temperature and physiological temperature. Moreover, a (first) degree of polymerization within this range may assist with attaining the desired thermoresponsive behavior, i.e. having a complex viscosity which increases with the temperature increase from room temperature to physiological temperature upon application of the composition within the oral cavity.

The aqueous topical oral composition may comprise a further poloxamer for increasing the complex viscosity of the composition at the physiological temperature relative to room temperature, the further poloxamer having a third degree of polymerization corresponding to the total number of monomer units of the further poloxamer. The further poloxamer may be included in the composition, for example, at a concentration of 0.1 to 1.5 wt.%, such as 0.2 to 1.0 wt.%, e.g. 0.25 to 0.85 wt.%.

Including such a further poloxamer in the composition may enable enhanced tuning of the viscosity and thermoresponsive behavior of the composition than, for instance, when only a single poloxamer is included in the composition. For example, addition of a further poloxamer whose viscosity switching temperature (defined as the temperature at the midpoint (50% of the viscosity difference) of the transition of the complex viscosity of the composition from the lower viscosity to the higher viscosity, as measured using a rheometer (Physica MCR 300) at 1Hz frequency)) is higher than that of the poloxamer may enable the resultant switching temperature of the composition to be increased, e.g. from 22.5°C to >25°C.

In some embodiments, the (third) degree of polymerization of the further poloxamer may be lower than the (first) degree of polymerization of the poloxamer and/or the fraction of ethylene oxide in the further poloxamer may be higher than the ethylene oxide content of the poloxamer.

When such a further poloxamer is included in the composition, the second degree of polymerization of the polysaccharide may, for example, be higher than the third degree of polymerization of the further poloxamer. The polysaccharide may therefore have chains which are longer or larger than those of both the poloxamer and the further poloxamer, which may assist in improving the dissolution resistance of the gelled composition via the previously described entanglement mechanism.

In an embodiment, the total poloxamer content of the composition is 8 to 30 wt.%, such as 10 to 25 wt.%, e.g. 12 to 18 wt.% of the composition. The composition may include a single poloxamer or more than one poloxamer type, as described in relation to the embodiment in which a further poloxamer is present in the composition. The total poloxamer content of the composition may account for all of the poloxamer(s) included in the composition. A total poloxamer content in this range may assist with providing a composition which is suitably thickened when applied within the oral cavity.

The polysaccharide may be substituted with at least one hydrophobic group. The hydrophobic groups may assist in the formation of networks in the gelled composition, which may further assist to increase the dissolution resistance of the gel. For example, the at least one hydrophobic group may include a methyl, an ethyl and/or a propyl group.

The degree of substitution of the polysaccharide with the hydrophobic group, as defined by the number of hydrophobic groups per monomer unit, may be, for example, 0.1 to 3, such as 0.5 to 2.5, e.g. 1.5 to 2.0, provide a further means of tuning the dissolution resistance or sustained release properties of the gel (when an active agent is included in the composition).

The (second) degree of polymerization of the polysaccharide may be in the range of 60 to 2000, such as 100 to 1000, e.g. 250 to 750. Such a degree of polymerization may mean that the polysaccharide is soluble in the composition, whilst having a sufficiently large molecular size to increase the dissolution resistance of the gelled composition. A relatively low (second) degree of polymerization, e.g. below about 200, may lead to a poorer dissolution resistance compared to polysaccharides having a higher (second) degree of polymerization, e.g. above about 200. However, such poorer dissolution resistance may be at least partially counteracted by increasing the concentration of the polysaccharide additive in the composition.

The percentage difference between the (first) degree of polymerization of the poloxamer and the (second) degree of polymerization of the polysaccharide may be 30 to 70%, such as 35 to 65%, e.g. 40 to 60%. This percentage difference may be equal to: (second degree of polymerization - first degree of polymerization)/second degree of polymerization.

The polysaccharide may be at least one selected from a cellulose derivative, chitosan, carrageenan, and xanthan gum. Such polysaccharides benefit from being non-toxic and readily available. Moreover, these polysaccharides are available with molecular weights which render them both soluble in the aqueous composition and having a larger (second) degree of polymerization than the (first) degree of polymerization of the poloxamer. The cellulose derivative may, for example, include a cellulose ether.

In an embodiment, the cellulose derivative includes, or is, at least one selected from hydroxypropyl methylcellulose and hydroxyethyl methylcellulose. These cellulose derivatives may themselves exhibit thermoresponsive behavior, and thus may advantageously assist with tuning the gelling or thickening properties of the composition, as well as enhancing the dissolution resistance of the compositions. In alternative embodiments, the polysaccharide additive exhibits negligible thermoresponsive behavior.

The aqueous topical oral composition may further comprise a surfactant. Whilst the poloxamer itself may inherently possess surfactant properties, including an additional dedicated surfactant in the composition may increase the shelf-life of the composition, e.g. by avoiding phase separation of the composition. Any suitable surfactant may be employed for this purpose. For example, the surfactant may be selected from one or more of a nonionic surfactant, an anionic surfactant, a cationic surfactant, and an amphoteric surfactant. The surfactant may, for instance, have a concentration in the composition of 0.5 to 5 wt.%, such as 1 to 4 wt.%, e.g. 1.5 to 3 wt.%. In a particular example, the surfactant is or includes a polysorbate surfactant, such as polyoxyethylene-sorbitan-20-monooleate, also known as Tween 80.

The composition may comprise an active agent. The active agent may, for example, comprise a tooth whitening agent and/or an antimicrobial agent. The antimicrobial agent may be or comprise, for instance, cetylpyridinium chloride. The tooth whitening agent may, for example, be or include a peroxide compound, such as hydrogen peroxide or carbamide peroxide.

Alternatively or additionally, the composition may include, as the active agent, one or more of chlorhexidine, a stannous salt, a zinc salt, amorphous calcium phosphate, a fluoride salt, one or more essential oils, triclosan, delmopinol, an antibiotic, a prebiotic, a probiotic, one or more vitamins, and an anti-oxidant.

The release of the active agent(s) from the gelled/thickened composition may be controlled by the polysaccharide additive included in the composition, as previously described. The active agent may be included in the composition in a concentration of 0.05 to 35 wt.%.

When an antimicrobial agent is included in the composition, the concentration of the antimicrobial agent may be 0.05 to 1.5 wt.%, such as 0.08 to 1.0 wt.%, e.g. 0.1 to 0.5 wt.% of the composition.

When a tooth whitening agent is included in the composition, e.g. a peroxide compound, the concentration of the tooth whitening agent may be, for example, 5 to 35 wt.%, such as 15 to 30 wt.%. When the whitening agent is, for example, hydrogen peroxide, the hydrogen peroxide may be included in the composition at a concentration of up to 25 wt.%. When the whitening agent is, for example, carbamide peroxide, the carbamide peroxide may be included in the composition at a higher concentration, such as up to 35 wt.%. The composition may, in some examples, include a mixture of hydrogen peroxide and carbamide peroxide.

More generally, it has been found from experiments in which various active agents, including different salts, and/or surfactants are included in the composition that the above-described dissolution resistance effect is exhibited regardless of the presence (or otherwise) of such species. Whilst the selection and amount of active agent and/or surfactant may exert at least some influence on the thermoresponsive behavior of the composition, by selection of the components and their concentrations from the above-provided ranges, the viscosity and thermoresponsive properties of the composition can be straightforwardly controlled, as will be described further herein below.

According to another aspect, there is provided a kit for preparing the aqueous topical oral composition as defined above. The kit may comprise one or more separately supplied components for combining together to form the composition prior to use, e.g. immediately prior to use.

When the composition includes an active agent, the poloxamer(s) and/or the polysaccharide additive may, in certain embodiments, be supplied separately from the active agent. The active agent may, for example, react relatively slowly with the poloxamer and/or the additive over time, such that supplying the active agent separately from either or both components may have shelf-life benefits. The composition can be prepared by mixing the components immediately prior to use, e.g. immediately prior to application of the composition within the oral cavity.

When, for example, a whitening agent is employed, the polysaccharide additive may exhibit at least some reactivity with the whitening agent, e.g. peroxide compound. The additive may be correspondingly supplied in the kit separately from the whitening agent.

The poloxamer, on the other hand, may be relatively stable in the presence of the whitening agent, e.g. peroxide compound, such that the poloxamer may be supplied premixed with the whitening agent in the kit.

According to still another aspect, there is provided an apparatus comprising: a supply of the aqueous topical oral composition as defined above; and an applicator for applying the aqueous topical oral composition within the oral cavity.

The above-described compositions were additionally found to display thixotropic behavior. In other words, the composition was found to have improved flowability when agitated. In an embodiment, the applicator includes an agitator for exerting a stress on the composition prior to application. The inclusion of such an agitator, such as a vibrating element, in the apparatus may enhance the ease with which the composition may be applied, and may permit the composition to be supplied with a higher room temperature complex viscosity than in the scenario in which no agitator is included in the apparatus.

The applicator may comprise a pump for pumping the aqueous topical oral composition from the supply to the oral cavity. The pump may be, for example, a manually operated pump or an electrically driven pump. The agitator may, for example, be included in the pump.

According to yet another aspect, there is provided a method comprising applying the aqueous topical oral composition as defined above within the oral cavity. The method may be therapeutic or non-therapeutic. Tooth whitening may be regarded, for example, as a non-therapeutic, e.g. cosmetic, method using the composition. In such an example, the composition may include a whitening agent, as previously described.

Therapeutic uses may, for example, include treatment of gingivitis, for example using an antimicrobial agent, as previously described.

In either case, the method may, for example, comprise applying the composition to a tooth, between teeth and/or the gums.

According to a further aspect, there is provided an aqueous topical oral composition as defined above for use in the treatment of an indication. The indication may be, for example, an oral health condition, such a gingivitis or periodontitis.

BRIEF DESCRIPTION OF THE DRAWINGS

Embodiments of the invention are described in more detail and by way of non-limiting examples with reference to the accompanying drawings, wherein:

FIG. 1 schematically depicts a pair of neighboring teeth and the gap therebetween;
FIG. 2 schematically depicts part of an apparatus for applying a topical oral composition;
FIG. 3 shows graphs of antimicrobial agent release versus time for five exemplary compositions;
FIG. 4 shows graphs of complex viscosity (log scale) versus temperature for the five exemplary compositions of FIG. 3;
FIG. 5 shows graphs of complex viscosity versus temperature for four exemplary compositions;
FIG. 6 shows the calculated results of a response optimizer (Minitab 18) maximizing the complex viscosity at 33°C and minimizing the complex viscosity at 25°C;
FIG. 7A shows a graph of complex viscosity versus frequency at room temperature (25°C) for a representative exemplary composition;
FIG. 7B shows a graph of complex viscosity versus temperature at 1Hz for the representative exemplary composition of FIG. 7A;
FIG. 7C shows a graph of complex viscosity versus time at 35°C and 1Hz for the representative exemplary composition of FIGs. 7A and 7B;
FIG. 8 shows graphs of antimicrobial agent release versus time for two exemplary compositions;

FIG. 9 shows a photograph of a typodont apparatus used to assess the antimicrobial performance of an exemplary composition;

FIG. 10 shows a photograph for explaining the split-mouth experimental design used to assess the antimicrobial performance of the exemplary composition referred to in relation to FIG. 9;

FIG. 11 shows photographs of the teeth and gums of each side of the mouth of the split-mouth design referred to in relation to FIG. 10;

FIG. 12 shows a bar chart of biofilm percentage for exemplary compositions and a control composition;

FIG. 13 schematically depicts an apparatus according to an example;

FIG. 14 depicts an apparatus according to another example being used to apply a composition between teeth; and

FIG. 15 schematically depicts an apparatus according to a further example.

## DETAILED DESCRIPTION OF THE EMBODIMENTS

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Provided is an aqueous topical oral composition. The composition is suitable for applying within the oral cavity, e.g. to teeth and/or gums. The composition comprises a poloxamer for increasing the complex viscosity of the composition at a physiological temperature, e.g. $\geq 33°C$, such as about $37°C$, which is higher relative to room temperature, e.g. $\leq 25°C$. The poloxamer thus thickens the composition upon application within the oral cavity, thereby improving its substantivity therein. The poloxamer has a degree of polymerization corresponding to the total number of monomer units of the poloxamer. The composition further includes an additive in the form of a polysaccharide. The additive is soluble in the composition at room temperature. It follows that the additive is also soluble in the composition at the physiological temperature. The polysaccharide has a degree of polymerization corresponding to the total number of monomer units of the polysaccharide. The degree of polymerization of the polysaccharide is higher than the degree of polymerization of the poloxamer. Further provided is a kit for preparing the composition, an apparatus for applying the composition, and methods comprising application of the composition within the oral cavity.

Thermoresponsive compositions which switch from fluidic at room temperature to a gel form in the oral cavity can be very useful to increase substantivity of active agents. Currently known compositions employed, for example, as whitening gels easily dissolve in the oral cavity, and therefore are not suitable for use without trays or lip retractors. Thermoresponsive compositions are disclosed herein which exhibit resistance under the conditions within the oral cavity for several hours, but are easily delivered using relatively low pressures due to their low viscosity at room temperature. The delivery can be further facilitated using vibrations, since the compositions are thixotropic.

Conventional poloxamer-containing oral compositions have been found to have disadvantageously low resistance to dissolution in saliva. This means that saliva flowing around the applied composition (in the form of a gel inside the oral cavity) causes the composition to be relatively quickly, e.g. within minutes, washed away. This observation led to such poloxamer-containing oral compositions being initially discarded for applications requiring longer term sustained release of active agents in the oral cavity.

A desirable characteristic of a topical oral composition is correspondingly relatively slow dissolution in saliva once applied within the oral cavity. The composition may thus remain applied for about 6 hours in an interdental space, when submersed in flowing liquid at $T \geq 33°C$.

Regarding this characteristic, the resistance of a poloxamer-containing aqueous topical oral composition to dissolution in saliva may be increased by including the polysaccharide additive in the composition. In particular, by the polysaccharide having a (second) degree of polymerization corresponding to the total number of monomer units of the polysaccharide which is higher than a (first) degree of polymerization of the poloxamer, the additive may decrease the solubility of the in situ gelled composition applied within the oral cavity. Without wishing to be bound by any particular theory, gelation of the poloxamer at the physiological temperature may coincide with the larger polysaccharide molecules providing entanglements within the gel, which entanglements restrict motion of the poloxamer molecules within the gel. The entanglements thereby reduce the aqueous solubility of the gelled composition relative to a composition which does not include such a polysaccharide additive.

This means that the gelled composition is less susceptible to being washed away by saliva, and therefore remains applied to a surface, e.g. on a tooth, between teeth or on gums, etc., for a longer period of time relative to a composition which does not include such an additive. The composition is correspondingly particularly useful for, for example, sustained release of an active agent included in the composition, e.g. over a number of hours, such as overnight.

The (second) degree of polymerization of the polysaccharide may be in the range of 60 to 2000. Such a degree of polymerization may mean that the polysaccharide is soluble in the composition, whilst having a sufficiently large molecular size to increase the dissolution resistance of the gelled composition.

The poloxamer may have a triblock copolymer structure having the general formula: $[CH_2CH_2O]_x[CH(CH_3)CH_2O]_y[CH_2CH_2O]_z$, and the first degree of polymerization may be the sum of x, y and z. The (first) degree of polymerization of the poloxamer may be determined using size exclusion chromatography. In particular, the number average molecular weight may be given by: Mn=∑i Ni Mi/(∑i Ni), where Ni=number of polymer species in fraction i, and Mi=molecular weight of polymer species in fraction i.

The following SEC conditions may be used: polyethylene glycol (PEG) calibration standards (Mp: 430, 982, 1960, 3020, 6690, 12300, 26100 and 44 000 g/mol); eluent: THF; flow rate: 1 ml/min; injection volume: 100 $\mu$l; column: particle size=5 $\mu$m, material=styrene-divinylbenzene; temperature: 40° C; detector: refractive index (RI).

The oxypropylene:oxyethylene ratio may be determined by NMR, per European Pharmacopoeia 5.0 (01/2005:1464). The Mn and the oxypropylene:oxyethylene ratio may then be used to calculate the degree of polymerization (x + y + z), i.e. the total number of ethylene oxide and propylene oxide units.

The Mn, and thus the degree of polymerization, of the polysaccharide may also be determined by SEC, using the same flow rate, temperature, and RI detector as for the Mn determination of the poloxamer, but using an aqueous eluent (0.1 M NaCl (aq)) and a column for aqueous SEC (OHpak SB-806M HQ).

The poloxamer and the additive may be separated from each other prior to analysing each component of the composition. Such a separation may be achieved by HPLC.

The additive is dissolved in the aqueous composition. The additive may thus be soluble in the composition at room temperature, and may have a relatively low concentration in the composition of 0.5 to 5 wt.%, such as 0.8 to 2.5 wt.%, e.g. 1 to 2 wt.%. It has been found that keeping the additive concentration within these ranges advantageously provides the above-described dissolution resistivity effect at the physiological temperature but assists to ensure a low enough viscosity at room temperature such that the composition is straightforward to apply, e.g. inject, within the oral cavity.

The polysaccharide may be substituted with at least one hydrophobic group. The hydrophobic groups may assist in the formation of networks in the gelled composition, which may further assist to increase the dissolution resistance of the gel. For example, the at least one hydrophobic group may include a methyl, an ethyl and/or a propyl group.

The degree of substitution of the polysaccharide with the hydrophobic group, as defined by the number of hydrophobic groups per monomer unit, may be, for example, 0.1 to 3, such as 0.5 to 2.5, e.g. 1.5 to 2.0, provide a further means of tuning the dissolution resistance or sustained release properties of the gel (when an active agent is included in the composition).

The polysaccharide may be at least one selected from a cellulose derivative, chitosan, carrageenan, and xanthan gum. Such polysaccharides benefit from being non-toxic and readily available. Moreover, these polysaccharides are available with molecular weights which render them both soluble in the aqueous composition and having a larger (second) degree of polymerization than the (first) degree of polymerization of the poloxamer. The cellulose derivative may, for example, include a cellulose ether.

In an embodiment, the cellulose derivative includes, or is, at least one selected from hydroxypropyl methylcellulose and hydroxyethyl methylcellulose. These cellulose derivatives may themselves exhibit thermoresponsive behavior, and thus may advantageously assist with tuning the gelling or thickening properties of the composition, as well as enhancing the dissolution resistance of the compositions. In alternative embodiments, the polysaccharide additive itself exhibits no or negligible thermoresponsive behavior.

A further desirable characteristic is resistance of fluidic forces exerted by the tongue or during drinking when in the gelled state at T ≥ 33°C. For this characteristic, an estimate can be of the minimum viscosity ($\mu$) value at T > 33°C required to resist drinking pressure, using the Poiseuille equation (Equation 1):

$$\mu = \frac{\Delta P \pi d^4}{128 LQ} \qquad \text{Equation 1}$$

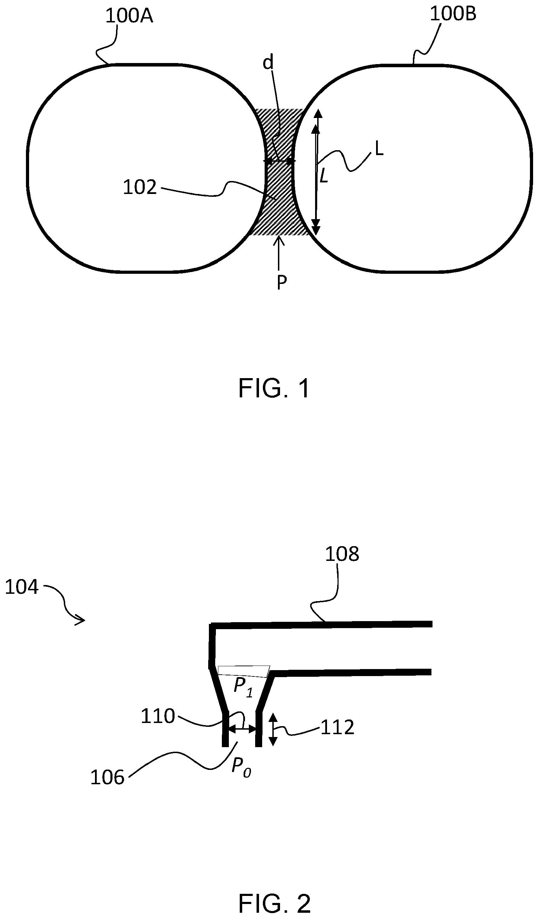

FIG. 1 schematically depicts a pair of teeth 100A, 100B and an interdental space 102 therebetween. The composition in its thickened/gelled state is harbored, in this explanatory example, in the interdental space 102. The arrow and the reference letter P shown in FIG. 1 represents the pressure being applied to gel composition residing in the interdental space 102. FIG. 1 also shows the width, d, and the length, L, of the interdental space 102. ΔP in the Poiseuille equation is the pressure difference over the interdental space 102 which harbours the gel, and Q is the maximum allowed flow rate of gel to maintain the gel in the interdental space 102.

Drinking pressures may peak on average at around 50 kPa, and in a worst case estimate half of that pressure may be directed on the interdental space, for a total of 5 seconds in one direction (upon moving the liquid around, the direction may fluctuate). A typical interdental space at the molar positions may harbour about 0.05 mL of gel. It is estimated that less than 10% of movement of the gel may be allowed in the 5 second period of peak pressure, and this would represent a flow rate of the gel of less than 0.001 mL/s. Based on these worst case estimates, and using Equation 1, the viscosity ($\mu$) of the gel at T $\geq$33°C may desirably be >123 Pa s.

It would also be desirable for the composition to be easy-to-deliver, e.g. through a nozzle, into the interdental space using a relatively low, e.g. manual, pushing force when the composition is at room temperature (T $\leq$25°C).

FIG. 2 schematically depicts part of an apparatus 104 for applying the composition within the oral cavity. In this particular non-limiting example, the composition is applied by ejection from a nozzle 106 into the interdental space 102. A conduit 108 may carry the composition from a supply or reservoir (not visible in FIG. 2) to the nozzle 106.

The diameter 110 and length 112 of the nozzle 106 are also shown in FIG. 2. A convenient pressure applied using a manual pump may be estimated to be about 0.5 bar. The above-described 0.05 mL dose of the composition to occupy the interdental space may be delivered in a relatively short time period, for example about 0.5 seconds. This means that the composition in this non-limiting example may be delivered at a flow rate of >0.1 mL/s.

The diameter 110 and length 112 of the nozzle 106 may be, for instance, 1.2 mm and 2 mm respectively. Such dimensions may be suitable for dosing the composition into the interdental space (these dimensions corresponding to those of the nozzle of the original AirFloss device marketed by Philips). In this example and using Equation 1, the viscosity at room temperature (T $\leq$25°C) may desirably be <12.7 Pa s. This room temperature complex viscosity may be regarded as a threshold viscosity defining an upper limit for easy pumpability.

The above-calculated complex viscosities of the composition at room temperature and at $\geq$33°C may correspond to suitable targeted viscosity properties of the composition.

In this respect, the first degree of polymerization of the poloxamer may be in the range of 50 to 300. A poloxamer having a (first) degree of polymerization within this range may assist with attaining a composition having the desired viscosity at room temperature and at physiological temperature. Moreover, a (first) degree of polymerization within this range may assist with attaining the desired thermoresponsive behavior, i.e. having a complex viscosity which increases with the temperature increase from room temperature to the physiological temperature upon application of the composition within the oral cavity.

In an embodiment, the total poloxamer content of the composition is 8 to 30 wt.%, such as 10 to 25 wt.%, e.g. 12 to 18 wt.% of the composition. A total poloxamer content in this range may assist with providing a composition which is suitably thickened when applied within the oral cavity.

The following is provided as a first set of non-limiting examples of the composition: B1-B4 (Table 1). The poloxamer employed in each of these examples is Poloxamer 407 (P407), which has a polypropylene oxide block having approximately 56 propylene oxide monomer units, and two polyethylene oxide blocks each having approximately 101 ethylene oxide monomer units. The (first) polymerization degree of P407 is therefore 258. P407 has an ethylene oxide content of about 78%.

The polysaccharide employed in each of B1-B4 is hydroxypropyl methylcellulose (HPMC). The HPMC had the following specifications:

Mn of about 120,000, giving a (second) degree of polymerization of about 600 ($\pm$10%), accounting for the following chemical composition data (using an average molecular weight per (six-membered ring) monomer of 180 to 187 g/mol).

Extent of labeling: 1.1-1.6 mol methoxy per mol cellulose (D.S.); 21 wt. % methoxy; 0.1-0.3 mol propylene oxide per mol cellulose (M.S.); 5 wt. % propylene oxide; surface tension: 43-55 dyn/cm, 25 °C, 0.05 wt. %; viscosity: 100,000 cP, 2 wt. % in $H_2O$(20 °C, Ubbelohde)(lit.); transition temp. gel point 70-90 °C.

The degree of substitution, taking both the methoxy and propoxy substituents into account, is 1.2 to 1.9.

Table 1.

| Name | Composition (wt.%) | | | Injectable? | Dissolution time in water | Dissolution time in artificial saliva |
|---|---|---|---|---|---|---|
| | Poloxamer (P407) | Polysaccharide additive (HPMC) | $H_2O$ | | | |
| B1 | 17 | 3 | 80 | No - but can be manipulated with spatula | >8h | Not tested |
| B2 | 17 | 1.5 | 81.5 | Yes | 2h10 | Not tested |
| B3 | 17 | 2 | 81 | Yes | 5h10 | Not tested |
| B4 | 17 | 2.5 | 80.5 | Yes | $\geq$8h | 5h35 |

Each of B1-B4 displayed a relatively long dissolution time in water of over 2 hours. The dissolution time increased as the concentration of HPMC increased, but it was observed that 3 wt.% of this particular additive resulted in a composition (B1) which was too viscous to inject using a syringe.

B4 exhibited a dissolution time of over five and a half hours in artificial saliva. This effect is attributed to the polysaccharide, in this case, HPMC, and is considered to derive from the entanglements in the gelled composition, as previously described. The dissolution times for these examples are thus significantly longer than for a composition which includes the poloxamer but no polysaccharide additive.

More generally, the composition may comprise an active agent. The active agent may, for example, comprise a tooth whitening agent and/or an antimicrobial agent. The antimicrobial agent may be, for instance, cetylpyridinium chloride. The tooth whitening agent may, for example, include a peroxide compound, such as hydrogen peroxide or carbamide peroxide.

Alternatively or additionally, the composition may include, as the active agent, one or more of chlorhexidine, a stannous salt, amorphous calcium phosphate, a fluoride salt, one or more essential oils, triclosan, delmopinol, an antibiotic, a prebiotic, a probiotic, one or more vitamins, and an anti-oxidant.

The release of the active agent(s) from the gelled/thickened composition may be controlled by the polysaccharide additive included in the composition. The active agent may be included in the composition in a concentration of 0.05 to 35 wt.%.

When an antimicrobial agent is included in the composition, the concentration of the antimicrobial agent may be 0.05 to 1.5 wt.%, such as 0.08 to 1.0 wt.%, e.g. 0.1 to 0.5 wt.% of the composition.

When a tooth whitening agent is included in the composition, e.g. a peroxide compound, the concentration of the tooth whitening agent may be, for example, 5 to 35 wt.%, such as 15 to 30 wt.%. When the whitening agent is, for example, hydrogen peroxide, the hydrogen peroxide may be included in the composition at a concentration of up to 25 wt.%. When the whitening agent is, for example, carbamide peroxide, the carbamide peroxide may be included in the composition at a higher concentration, such as up to 35 wt.%. The composition may, in some examples, include a mixture of hydrogen peroxide and carbamide peroxide.

When the composition includes a whitening agent, such as a peroxide compound, the active agent may further include one or more agents for reducing discomfort, e.g. in the form of a nitrate and/or phosphate salt. One or more fluoride salts may also be included in the composition. The composition may also include a flavoring and/or sweetener.

When the composition includes an antimicrobial agent, such as cetylpyridinium chloride, it has been found that the antimicrobial activity of the composition may be improved by including a fluoride salt and/or a zinc salt in the composition. This improvement in antimicrobial activity is as compared to a composition including the antimicrobial agent but neither a fluoride salt nor a zinc salt. The fluoride salt may be, for instance, sodium fluoride. The zinc salt may be, for example, zinc gluconate.

The fluoride salt may, for instance, be included in a concentration of 0.01 to 1 wt.%, e.g. about 0.03 to 0.20 wt.%, of the composition. The molar concentration of fluoride ions may be, for example, 10 to 90 mM, such as 15 to 85 mM.

The zinc salt may, for example, be included in a concentration of 0.1 to 5.0 wt.%, such as 0.5 to 4.0 wt.%., e.g. about 0.75 to 3.5 wt.%, of the composition. The molar concentration of zinc ions may be, for example, 5 to 30 mM, such as 10 to 20 mM, e.g. about 15 mM.

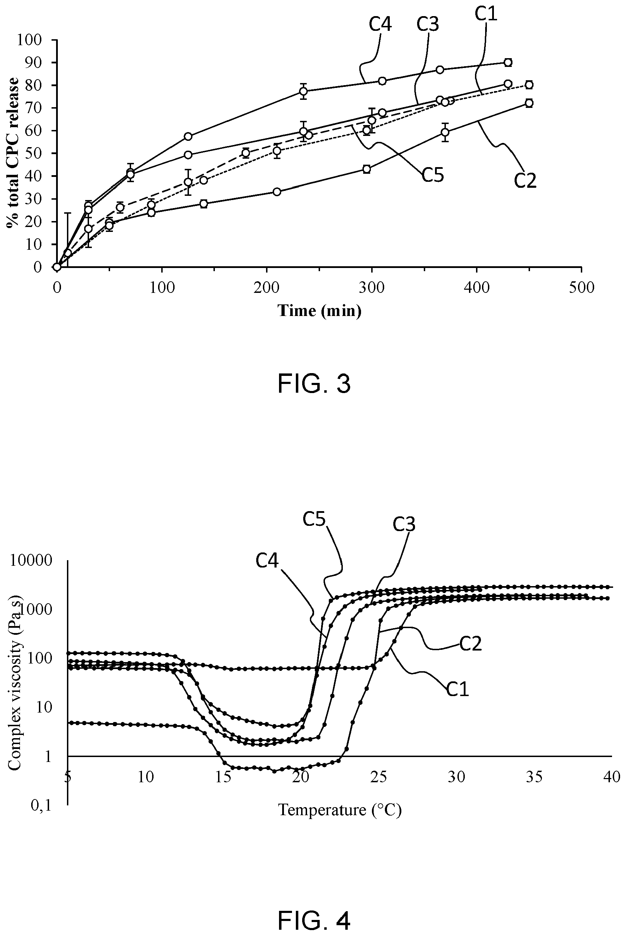

To study the release of an active agent from the gels, 0.1 wt.% of cetylpyridinium chloride (CPC) was included in the composition. FIG. 3 shows graphs of antimicrobial agent release versus time for five compositions, which can be regarded as a second set of non-limiting examples. Table 2 provides details of these compositions.

Table 2.

| Name | Composition (wt.%) | | | |
|------|---------------------|---------------------------------|-------------------|--------|
| | Poloxamer (P407) | Polysaccharide additive (HPMC) | Active agent (CPC) | $H_2O$ |
| C1 | 13 | 2.5 | 0.1 | 84.4 |
| C2 | 14 | 2.5 | 0.1 | 83.4 |
| C3 | 15 | 2.5 | 0.1 | 82.4 |
| C4 | 16 | 2.5 | 0.1 | 81.4 |
| C5 | 17 | 2.5 | 0.1 | 80.4 |

The poloxamer (P407) and the polysaccharide additive (HPMC) were the same in C1-C5 as in B1-B4. FIG. 3 shows the release profiles of each of C1-C5 in a 50 mM NaCl solution at 37°C. The 50 mM NaCl solution was selected due to having similar ionic strength to saliva. FIG. 3 shows that each of the compositions C1-C5 were found to be very suitable for a continuous release of an active agent, in this case CPC, over a 7 hour period. The whole tested range of 13 to 17 wt.% of the poloxamer (in this case P407) is suitable for sustained release of the active agent. In these particular examples, CPC is released continuously over 7 hours, which enables a relatively long plaque growth suppression, owing to the antimicrobial properties of CPC.

FIG. 4 shows graphs of complex viscosity (log scale) versus temperature for each of the compositions C1-C5. The switching temperature of each composition was tested using a rheometer (Physica MCR 300) at 1Hz frequency. C2-C5 were each found to have a room temperature ($\leq$25°C) complex viscosity of <12.7 Pa s, and physiological temperature ($\geq$33°C) complex viscosity of >123Pa s. Each of C2-C5 therefore have properties which make them particularly suitable for use as a topical oral composition.

The aqueous topical oral composition may comprise a further poloxamer for increasing the complex viscosity of the aqueous composition at the physiological temperature relative to room temperature; the further poloxamer having a third degree of polymerization corresponding to the total number of monomer units of the further poloxamer.

In a third set of non-limiting examples E1-E4 (Table 3), P407 was combined with Poloxamer 188 (PI88) in the composition. P188 exhibits a higher switching temperature than P407. P188 has a polypropylene oxide block having approximately 27 propylene oxide monomer units, and two polyethylene oxide blocks each having approximately 80 ethylene oxide monomer units. The (first) polymerization degree of P188 is therefore 187. P188 has an ethylene oxide content of about 86%.

Table 3.

| Name | Composition (wt.%) | | | | |
| | Poloxamer (P407) | Further Poloxamer (P188) | Polysaccharide additive (HPMC) | Active agent (CPC) | H$_2$O |
| --- | --- | --- | --- | --- | --- |
| E1 | 14 | 0 | 2 | 0.1 | 83.9 |
| E2 | 14 | 0.3 | 2 | 0.1 | 83.6 |
| E3 | 14 | 0.5 | 2 | 0.1 | 83.4 |
| E4 | 14 | 0.8 | 2 | 0.1 | 83.1 |

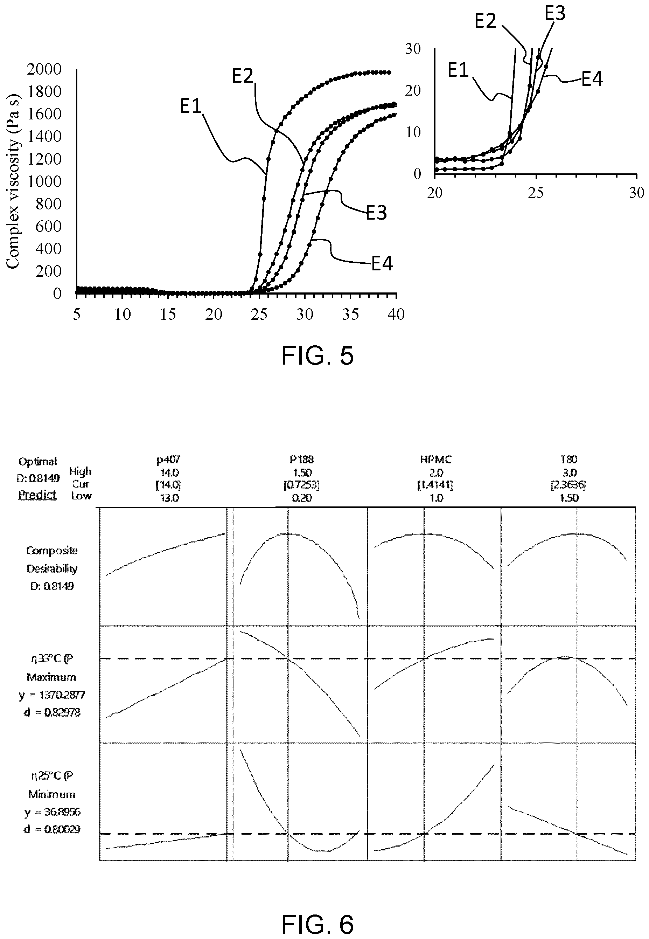

The polysaccharide additive (HPMC) and the active agent (CPC) were the same in E1-E4 as in C1-C5. FIG. 5 shows graphs of complex viscosity (at 1Hz) versus temperature for E1-E4. These examples show that addition of relatively small quantities of the further poloxamer (P188) increases the switching temperature of the composition (see the inset of FIG. 5 which provides a zoomed-in view of the switching temperature region of the curves), although at 25°C the viscosity still exceeds <12.7 Pa s.

In E1-E4, the polysaccharide additive (HPMC) concentration was lowered (relative to that in C1-C5) to 2 wt.%, in order to compensate for the increased low T viscosity resulting from the addition of the further poloxamer (P188).

More generally, the further poloxamer may be included in the composition at a concentration of 0.1 to 1.5 wt.%, such as 0.2 to 1.0 wt.%, e.g. 0.25 to 0.85 wt.%. Including such a further poloxamer in the composition may enable enhanced tuning of the viscosity and thermoresponsive behavior of the composition, than, for instance, when only one poloxamer is included in the composition. The further poloxamer may, for example, have a lower (third) degree of polymerization than the (first) degree of polymerization of the poloxamer. Alternatively or additionally, the further poloxamer may, for example, have a higher ethylene oxide content than that of the poloxamer.

When such a further poloxamer is included in the composition, the second degree of polymerization of the polysaccharide may, for example, be higher than the third degree of polymerization of the further poloxamer. The polysaccharide may therefore have chains which are longer or larger than those of both the poloxamer and the further poloxamer, which may assist in improving the dissolution resistance of the gelled composition via the previously described entanglement mechanism.

The aqueous topical oral composition may further comprise a surfactant. Whilst the poloxamer itself may inherently possess surfactant properties, including an additional dedicated surfactant in the composition may increase the shelf-life of the composition, e.g. by avoiding phase separation of the composition. Any suitable surfactant may be employed for this purpose. For example, the surfactant may be selected from one or more of a nonionic surfactant, an anionic surfactant, a cationic surfactant, and an amphoteric surfactant. The surfactant may, for instance, have a concentration in the composition of 0.5 to 5 wt.%, such as 1 to 4 wt.%, e.g. 1.5 to 3 wt.%.

In a particular non-limiting example, the surfactant is or includes a polysorbate surfactant, such as polyoxyethylene-sorbitan-20-monooleate, also known as Tween 80. This was found to be particularly effective in preventing the compositions from separating into a solid and fluidic phase over time, e.g. after several weeks.

The surfactant may nevertheless exert some influence on the viscosity profiles of the compositions in which it is included. Table 4 summarizes a designed experiment (DoE) for optimizing the complex viscosities of the compositions at 25°C and 33°C.

Table 4.

| Input formulation parameters | Values |
|---|---|
| P407 (wt.%) | 13; 13.5; 14 |
| P188 (wt.%) | 0.2; 0.4; 0.8; 1; 1.2; 1.5 |
| HPMC (wt.%) | 1; 1.5; 2 |
| T80 (wt.%) | 1.5; 2; 3 |
| CPC (wt.%) | 0.1 |
| **Critical to Quality Outputs (CTQs)** | |
| Viscosity of fluid at 25 °C (Pa s) | |
| Viscosity of fluid at 33 °C (Pa s) | |

FIG. 6 shows the analysis of the response optimizer in Minitab 18 maximizing the viscosity at 33°C and minimizing the viscosity at 25°C. The numbers in parentheses in FIG. 6 show the optimal composition arising from the DoE: 14.0 wt.% poloxamer (P407), 0.73 wt.% further poloxamer (P188), 1.41 wt.% polysaccharide additive (HPMC), and 2.36 wt.% surfactant (Tween 80).

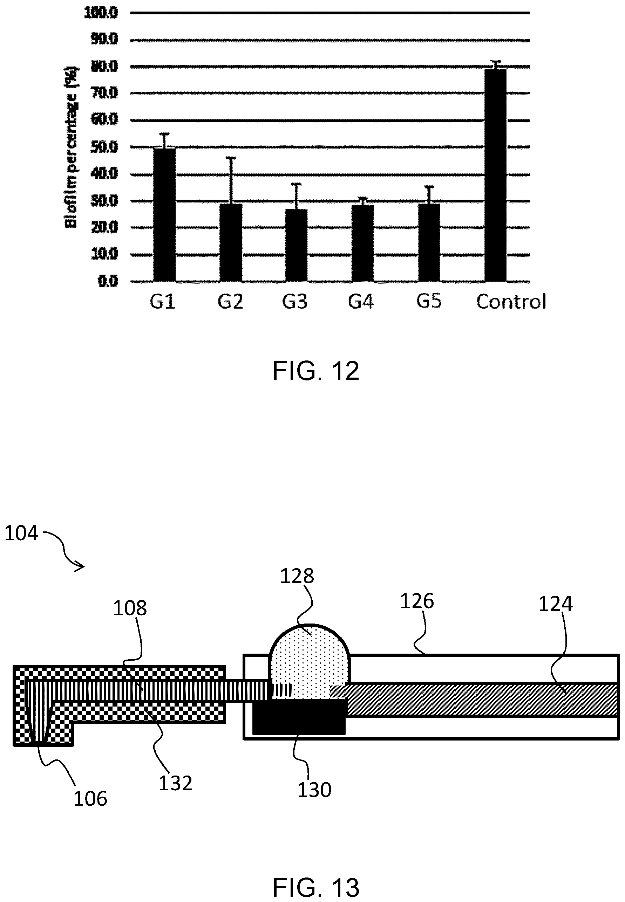

It is also evident from FIG. 6 that the predicted viscosity at 25°C of the optimal composition is close to 37 Pa s, so larger than 12.7 Pa s (see the above discussion in relation to FIG. 2). The target viscosity may nevertheless be adapted by choosing larger nozzle diameters, or by increasing the delivery pressure, which may require greater pump power, or more manual power. Embodiments of the apparatus 104 for applying the composition will be described in more detail herein below with reference to FIGs. 13-15.

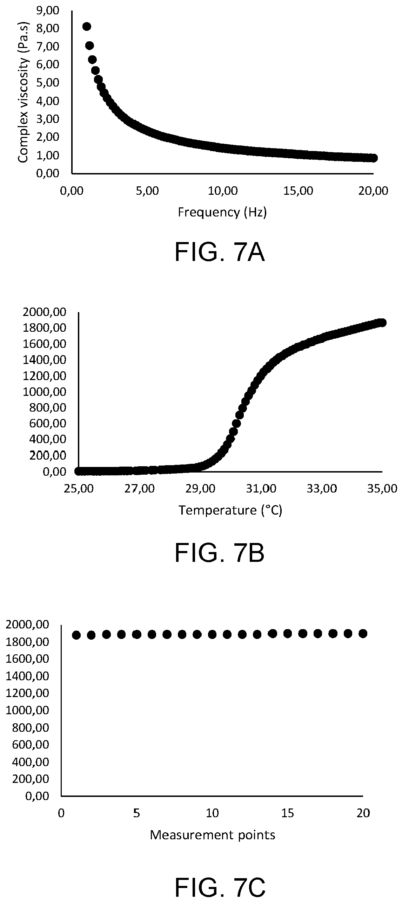

FIGs. 7A-7C provide a more complete viscosity test on one of the compositions of the designed experiment: 14 wt.% poloxamer (P407), 0.4 wt.% further poloxamer (PI88), 1 wt.% polysaccharide additive (HPMC), 3 wt.% surfactant (Tween 80), 0.1 wt.% active agent (CPC), and 81.5 wt.% water.

The composition was first subjected to a frequency sweep at room temperature (25°C), and FIG. 7A shows a graph of complex viscosity versus frequency. This rheological experiment is for mimicking the stresses on the composition during delivery/application to the oral cavity. FIG. 7A shows the thixotropic nature of the composition, which is generally representative of the compositions described herein.

FIG. 7B shows a graph of complex viscosity versus temperature at 1Hz. This experiment is for mimicking the temperature increase once the composition has been applied within the oral cavity. FIG. 7B shows the complex viscosity increasing as the temperature increases towards physiological temperature ($\geq$33°C).

FIG. 7C shows a graph of complex viscosity versus time at 35°C and 1Hz. This experiment shows the stability of the viscosity at elevated temperature following the temperature increase shown in FIG. 7B.

A fourth set of exemplary compositions were prepared in order to verify that the composition including the surfactant and the further poloxamer still provided sustained release of an active agent. Table 5 provides details of the non-limiting exemplary compositions F1 and F2.

Table 5.

| Name | Composition (wt.%) | | | | | |
|---|---|---|---|---|---|---|
| | Poloxamer (P407) | Further Poloxamer (P188) | Polysaccharide additive (HPMC) | Surfactant (Tween 80) | Active agent (CPC) | $H_2O$ |
| F1 | 14 | 0.4 | 1 | 3 | 0.1 | 81.5 |
| F2 | 14 | 1.5 | 1.5 | 2 | 0.1 | 80.9 |

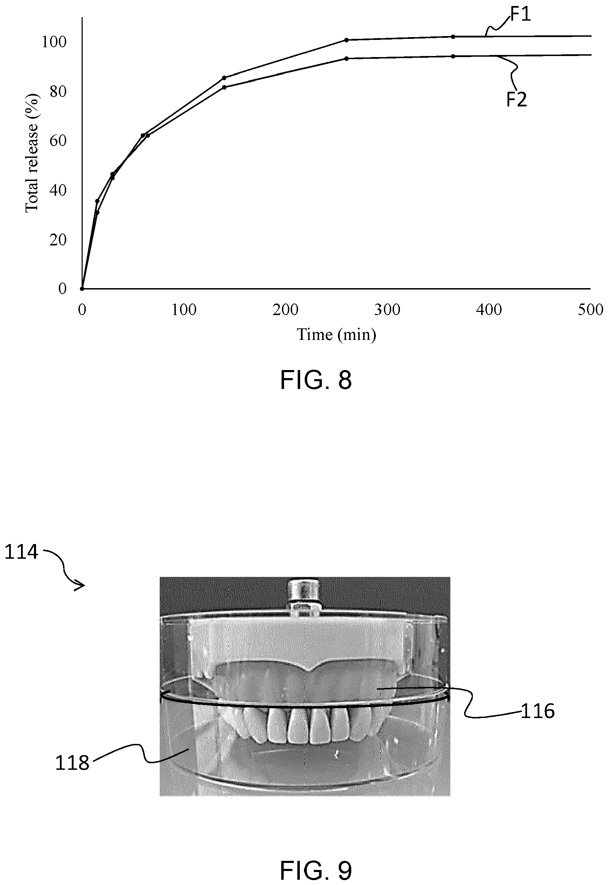

FIG. 8 shows graphs of active agent (CPC) release versus time for F1 and F2. Whilst the release of the active agent was slightly faster than, for example, C1-C5 (see Fig. 3), the release observed with F1 and F2 still took about 3 hours to reach completion. This particular example represents a significantly longer CPC antimicrobial treatment time, especially in comparison to conventional CPC mouthwashes, where the CPC is washed away by saliva within minutes.

FIG. 9 shows a photograph of a typodont apparatus 114 used to assess the antimicrobial performance of an exemplary composition. Antimicrobial activity of the compositions was tested on saliva-based plaque grown on a typodont 116. The typodont 116 was suspended in artificial saliva growth medium 118 inoculated with collected human saliva. A plaque biofilm was allowed to grow on the typodont 116 for 7 hours at 37°C.

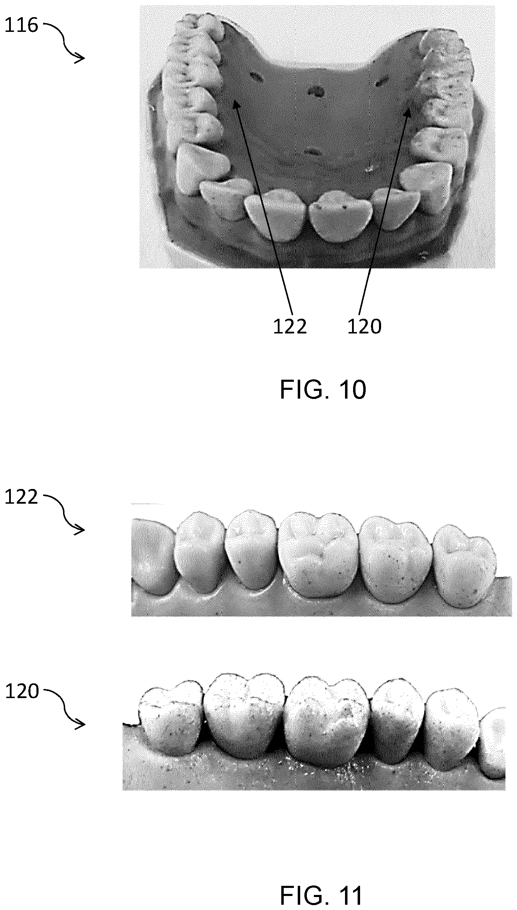

FIG. 10 shows a photograph for explaining the split-mouth experimental design used to assess the antimicrobial performance of the composition following growth of the plaque biofilm. A commercially available mouthwash containing CPC was injected into the interdental spaces on a first side 120 of the typodont 116. One of the compositions according to the present disclosure was injected into the interdental spaces on a second side 122 of the typodont 116. The typodont 116 was kept warm while the mouthwash and the composition were applied thereto. The typodont 116 was then re-immersed in fresh artificial saliva at 37°C, and incubated for another 40 hours.

FIG. 10 shows the typodont 116 following the incubation. It is evident from FIG. 10 that the first side 120 to which the mouthwash was applied had greater plaque buildup than the second side 122 to which the composition according to the present disclosure was applied. This is more clearly evident in FIG. 11, which shows the second side 122 (upper pane) and the first side 120 (lower pane). This effect is partly ascribed to the relatively high dissolution resistance of the compositions according to the present disclosure, since the polysaccharide additive helps to prevent the poloxamer-containing composition from being washed away by the saliva.

As a further comparative example, poloxamer (P407) solutions without any polysaccharide additive were injected into the interdental spaces of a similar typodont model, which was placed at 37°C in a beaker with water or artificial saliva. The water or artificial saliva was stirred using a stirring bar. The pure poloxamer solutions dissolved within minutes in both aqueous media.

A fifth set of exemplary compositions were prepared in order to verify that the composition can be generally used to effect sustained release of various active agents. Table 6 provides details of the non-limiting exemplary compositions G1-G5.

Table 6.

| Name | Composition (wt.%) | | | | | | |
|---|---|---|---|---|---|---|---|
| | Poloxamer (P407) | Further Poloxamer (P188) | Polysaccharide additive (HPMC) | Surfactant (Tween 80) | Active agent (CPC) | H$_2$O | Other |
| **G1** | 14 | 1.5 | 1.5 | 2 | 0.1 | 80.9 | - |
| **G2** | 14 | 1.5 | 1.5 | 2 | 0.1 | 80.6 | 0.3% sodium fluoride |
| **G3** | 14 | 1.5 | 1.5 | 2 | 0.1 | 80.15 | 0.75% zinc gluconate |
| **Control** | 14 | 1.5 | 1.5 | 2 | - | 81 | - |
| **G4** | 14 | 1.5 | 1.5 | 2 | 0.1 | - | 80.9% Perioshield |
| **G5** | 14 | 1.5 | 1.5 | 2 | - | - | 81% Perioshield |

G2 and G3 additionally contain 0.3% NaF, and 0.75% zinc gluconate respectively. These produced the desired gelling and resistance to dissolution, and also showed improved antimicrobial effects over G1, which just contained CPC as the active agent. This shows that even with additional ionic components, the desirable effects of the composition are still exhibited. A commercial mouthwash (Perioshield) was also added to the composition (G4 and G5), which mouthwash contains flavors and an alternative antimicrobial to CPC. These compositions were also highly efficacious in suppressing biofilm growth, as shown in FIG. 12.

Whilst in the above-described investigations, CPC was used as the active agent, the composition according to the present disclosure may also be employed with various alternative active agents. Hydrogen peroxide is often formulated with poloxamers, so may correspondingly be compatible with the composition. Additionally, many active agents useful for many different applications may also be used, such as chlorhexidine, stannous salts, zinc salts, amorphous calcium phosphate, fluorides, essential oils, triclosan, delmopinol, antibiotics, prebiotics, probiotics, vitamins, anti-oxidants, etc.

In some embodiments, the composition is provided as a kit for preparing the aqueous topical oral composition described above. The kit may comprise one or more separately supplied components for combining together to form the composition prior to use, e.g. immediately prior to use.

When the composition includes an active agent, the poloxamer(s) and/or the polysaccharide additive may be supplied separately from the active agent. The active agent may, for example, react relatively slowly with the poloxamer and/or the additive over time, such that supplying the active agent separately from either or both components may have shelf-life benefits. The composition can be prepared by mixing the components immediately prior to use, e.g. immediately prior to application of the composition within the oral cavity.

When, for example, a whitening agent is employed, the polysaccharide additive may exhibit at least some reactivity with the whitening agent, e.g. peroxide compound. The additive may be correspondingly supplied in the kit separately from the whitening agent.

The poloxamer, on the other hand, may be relatively stable in the presence of the whitening agent, e.g. peroxide compound, such that the poloxamer may be supplied premixed with the whitening agent in the kit.

More generally, the kit may be implemented in any suitable manner. For example, the separately supplied components may be contained in respective barrels of a multi-barrel syringe, in separate containers or vials, etc. When the kit comprises a multi-barrel syringe, the composition may be formed, for example, in a mixing tip of the syringe which receives the respective components from the barrels.

The present disclosure further provides an apparatus 104 comprising: a supply of the aqueous topical oral composition as defined above; and an applicator for applying the aqueous topical oral composition within the oral cavity.

The apparatus 104 may, for example, comprise one or more of an electric flossing device, an irrigator, a toothbrush (e.g. a toothbrush having a vibrating brush-head), and a mouthguard. The apparatus 104 may, in some embodiments, take the form of a syringe; the barrel of the syringe defining the composition supply/reservoir, and the outlet of the syringe defining the applicator.

The above-described compositions were additionally found to display thixotropic behavior. In other words, the compositions were found to have improved flowability when agitated. As shown in FIG. 7A, the frequency sweep at 25°C results in a decrease in the viscosity of approximately 4 times at 5Hz and 8 times at 20 Hz. This brings the composition easily within the desired range of viscosity during delivery/application. The numerous exemplary compositions which were tested all showed a similar thixotropic response.

FIG. 13 schematically depicts an apparatus 104 according to a non-limiting example. The apparatus 104 includes a nozzle 106 for applying the composition within the oral cavity, e.g. to teeth and/or gums. The composition is stored in a reservoir 124 and is supplied to the nozzle 106 via the conduit 108.

The reservoir 124 may, for example, take the form of a cartridge or sachet which may be inserted, e.g. removably installed, into the apparatus 104. In the particular example shown in FIG. 13, the reservoir 124 is accommodated in a handle 126 of the apparatus 104. The cartridge or sachet may be replaced when the composition contained therein has been used up.

In an embodiment, the apparatus 104 includes an agitator 128, 130 for exerting a stress on the composition prior to its application within the oral cavity. The inclusion of such an agitator 128, 130 in the apparatus 104 may enhance the ease with which the composition may be applied, and may permit the composition to have a higher room temperature complex viscosity than in the scenario in which no agitator 128, 130 is included in the apparatus 104. Thus, the agitator 128, 130 may assist in dispensing the thixotropic composition.

The apparatus 104 may comprise a pump 128 for pumping the aqueous topical oral composition from the reservoir 124 to the oral cavity via the nozzle 106. The pump 128 may be, for example, a manually operated pump or an electrically driven pump. The pump 128 may thus be included in the agitator 128, 130. As shown in FIG. 13, the pump 128 may also be housed within the handle 126 of the apparatus 124.

In certain embodiments, the pump 128 includes a relatively low power electrically driven pump, such as a membrane pump. Such pumps may operate at frequencies above 10 Hz, thereby automatically reducing the viscosity of the composition as the composition is being pumped from the reservoir 124 to the oral cavity.

Alternatively or additionally, the agitator 128, 130 may include a vibrator 130 for applying a stress to the composition as it is being dispensed. The frequency of the vibrations provided by the vibrator 130 and/or by a vibrational pumping mode of the pump 128 may be sufficiently high to decrease the room temperature viscosity of the composition. The agitator 128, 130 may, for instance, enable the room temperature viscosity to be reduced 4 to 8 fold at frequencies of 5 to 20Hz.

The apparatus 104 may benefit from lower cost and enhanced user control when a manual, e.g. finger-operated, pump 128 is employed to dispense the composition. In particular, a finger-operated manual pump 128 may permit full user-control of the amount of the composition which is deposited in the oral cavity. In such a scenario, provision of a vibrator may advantageously assist with reducing the viscosity of the composition during application.

In an embodiment, the apparatus 104 may take the form of a toothbrush having an integrated vibrator 130 for vibrating the brush-head of the toothbrush. In such an example, the vibrator 130 may serve the dual purpose of improving the cleaning capability of the toothbrush and enabling easier manual pumping of the composition.

Alternatively, when no integrated vibrator is included in the apparatus 104, a vibrator 130 may simply be added, e.g. clipped, to the apparatus 104. In such an example, the vibrator 130 may, for instance, take the form of a relatively inexpensive vibrating excenter motor.

In the example shown in FIG. 13, the apparatus 104 comprises thermal insulation 132 around the nozzle 106 and/or conduit 108. The thermal insulation 132 may comprise an insulating foam, a vacuum jacket or a gas-filled pocket. The thermal insulation 132 may assist to avoid that the nozzle 106, which may be at least partially within the oral cavity during application, heats up and thereby thickens the composition before it has been applied. Thus, the thermal insulation 132 may assist to avoid such premature thickening of the composition within the nozzle 106, which may otherwise impede delivery of the composition.

As shown in FIG. 13, the thermal insulation 132 may conform to the tapering shape of the nozzle 106, which tapering shape may enable the nozzle 106 together with the thermal insulation 132 to conform to the interdental topography. This may assist to ensure optimal delivery of the composition into the interdental spaces 102.

Alternatively or additionally, a phase change material may surround the nozzle 106; the phase change material having a melting temperature of about 25°C. Such a phase change material may assist to delay any temperature increase beyond 25°C for a long enough time to deliver the composition into the oral cavity. The phase change material may, for example, be provided as a layer at least partially surrounding the nozzle 106 and/or conduit 108. Alternatively, the phase change material may occupy voids in a porous material at least partially surrounding the nozzle 106 and/or conduit 108.

In a more elaborate example, the apparatus 104 may comprise a cooling element (not shown) for cooling the composition prior to application. Whilst more expensive to incorporate in the apparatus 104, the cooling element may provide a more robust way of minimizing premature thickening of the composition within the apparatus 104 prior to delivery into the oral cavity. The cooling element may, for example, include a Peltier element.

The cooling may, in some examples, be provided to the walls delimiting the fluidic system which carries the composition from the reservoir 124 to the oral cavity. The reservoir 124 itself may alternatively or additionally be cooled by the cooling element. Such a cooling element may assist the apparatus 104 to be utilized in warm environments, such as in tropical environments, where bathroom temperatures may exceed 25°C.

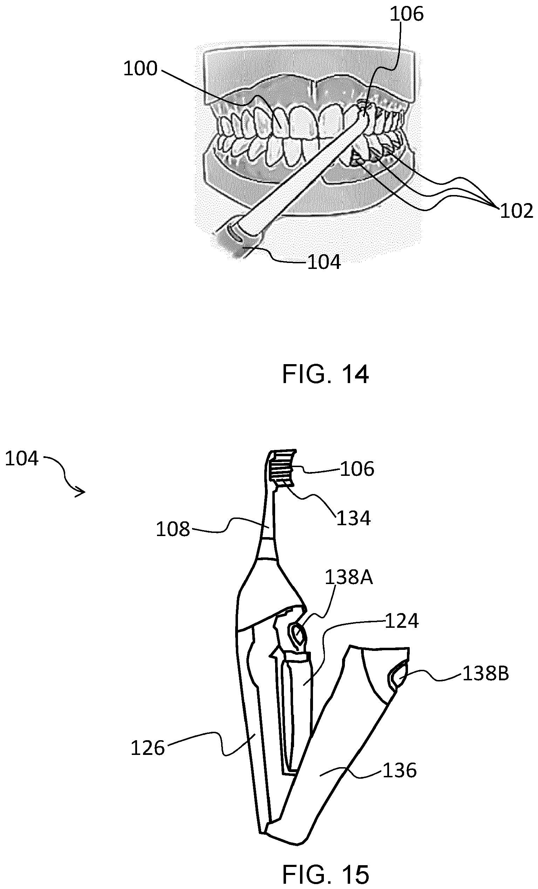

FIG. 14 depicts an apparatus 104 according to another example. In FIG. 14, the apparatus 104 is shown applying the composition in the interdental spaces 102 between the teeth 100 via the nozzle 106. The apparatus 104 may be configured to supply the composition in a stream of air to the interdental spaces 102, as per the AirFloss device marketed by Philips.

Since the compositions of the present disclosure may exhibit limited resistance to large shear forces exerted, for example, by the tongue, the substantivity of the composition on the exposed surfaces of the teeth 100 may be shorter than in the interdental spaces 102. This may mean that the compositions may be more usefully employed within the interdental spaces 102, which are more secluded and in which the tongue may have no or limited access. Since also oral disease is most prevalent in the interdental spaces 102, the composition according to the present disclosure may be usefully and valuably applied in such interdental spaces 102.

In an embodiment, the apparatus 104 comprises a resilient ring (not shown) extending around the exit of the nozzle 106. The resilient ring may assist to guide the composition into the interdental spaces 102, and thereby minimizes or prevents spilling of the composition on the exposed surfaces of the teeth 100. In other words, avoid the resilient ring may assist to ensure that more of the composition is pushed into or through the interdental space 102 and less is deposited on the outside of the teeth 100. The resilient ring may be fabricated from, for example, a soft silicone rubber.

FIG. 15 schematically depicts an apparatus 104 according to a further example. In this example, the apparatus 104 takes the form of a toothbrush having a brush-head 134. The composition is contained in a reservoir 124, taking the form of a removable sachet or cartridge, in the handle 126 of the toothbrush. As shown in FIG. 15, the front handle cover 136 of the toothbrush may be at least partially detachable, thereby to enable the sachet or cartridge to be replaced.

As shown in FIG. 15, the apparatus 104 may include a button arrangement 138A, 138B which operates a manual pump (not visible) for manually pumping the composition from the reservoir 124 through the core 106 of the brush-head stem. The core 106 of the brush-head stem thus defines a fluid channel. Such a pumping principle may be additionally employed to supply toothpaste to the brush-head 134, as per the IntelliClean system marketed by Philips.

In an embodiment, the apparatus 104 includes a vibrator (not visible in FIG. 15) for vibrating the brush-head 134. The vibrator may, for example, vibrate the brush-head at about 260 Hz, thereby causing the viscosity of the composition to decrease. In this manner, manual pumping of the composition to the brush-head 134 may be facilitated. Such a vibrating brush-head may, for instance, be implemented in a similar manner as in the Sonicare power toothbrush marketed by Philips.

Also provided is a method comprising applying the aqueous topical oral composition as defined above within the oral cavity. The method may be therapeutic or non-therapeutic. Tooth whitening may be regarded, for example, as a non-therapeutic, i.e. cosmetic, method using the composition. In such an example, the composition may include a whitening agent, as previously described.

Therapeutic uses may, for example, include treatment of gingivitis, for example using an antimicrobial agent, as previously described.

In either case, the method may, for example, comprise applying the composition to a tooth, between teeth and/or the gums.

Further provided is an aqueous topical oral composition as defined above for use in the treatment of an indication. The indication may be, for example, an oral health condition, such a gingivitis or periodontitis.

Whilst having been described in relation to topical oral applications, the present disclosure is also envisaged for further applications. For example, the composition of the present disclosure may be applied in advanced drug delivery. As well as being applied orally, the composition may be employed for application in or to other body parts, e.g. subcutaneous delivery by injection with a syringe needle.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

**Claims**

1. An aqueous topical oral composition comprising:

   a poloxamer for increasing the complex viscosity of the composition at a physiological temperature relative to room temperature, the poloxamer having a first degree of polymerization corresponding to the total number of monomer units of the poloxamer; and

   an additive in the form of a polysaccharide dissolved in the composition, the polysaccharide having a second degree of polymerization corresponding to the total number of monomer units of the polysaccharide which is higher than the first degree of polymerization of the poloxamer.

2. The aqueous topical oral composition according to claim 1, wherein the poloxamer has a triblock copolymer structure having the general formula:

$$[CH_2CH_2O]_x[CH(CH_3)CH_2O]_y[CH_2CH_2O]_z,$$

   and wherein the first degree of polymerization is the sum of x, y and z.

3. The aqueous topical oral composition according to claim 1 or claim 2, wherein the first degree of polymerization of the poloxamer is in the range of 50 to 300.

4. The aqueous topical oral composition according to any of claims 1 to 3, comprising a further poloxamer for increasing the complex viscosity of the composition at the physiological temperature relative to room temperature, the further poloxamer having a third degree of polymerization corresponding to the total number of monomer units of the further poloxamer; optionally wherein the second degree of polymerization of the polysaccharide is higher than the third degree of polymerization.

5. The aqueous topical oral composition according to any of claims 1 to 4, wherein the total poloxamer content of the composition is 8 to 30 wt.% of the composition.

6. The aqueous topical oral composition according to any of claims 1 to 5, wherein the polysaccharide is substituted with at least one hydrophobic group; optionally wherein the at least one hydrophobic group includes a methyl, an ethyl and/or a propyl group.

7. The aqueous topical oral composition according to any of claims 1 to 6, wherein the second degree of polymerization of the polysaccharide is in the range of 60 to 2000.

8. The aqueous topical oral composition according to any of claims 1 to 7, wherein the polysaccharide is at least one selected from a cellulose derivative, chitosan, carrageenan, and xanthan gum.

9. The aqueous topical oral composition according to claim 8, wherein the cellulose derivative includes at least one selected from hydroxypropyl methylcellulose and hydroxyethyl methylcellulose.

10. The aqueous topical oral composition according to any of claims 1 to 9, wherein the concentration of the additive in the composition is 0.5 to 5 wt.%.

11. The aqueous topical oral composition according to any of claims 1 to 10, further comprising a surfactant; optionally wherein the composition comprises 0.5 to 5 wt.% of the surfactant.

12. The aqueous topical oral composition according to any of claims 1 to 11, wherein the composition comprises an active agent; optionally wherein the active agent is a tooth whitening agent and/or an antimicrobial agent.

13. A kit for preparing the aqueous topical oral composition according to claim 12, wherein the poloxamer and/or the polysaccharide additive is or are supplied separately from the active agent.

14. An apparatus comprising:

a supply of the aqueous topical oral composition according to any of claims 1 to 12; and
an applicator for applying the aqueous topical oral composition within the oral cavity; optionally wherein the applicator comprises an agitator for exerting a stress on the composition prior to its application.

15. A method comprising applying the aqueous topical oral composition according to any of claims 1 to 12 within the oral cavity.

16. The method according to claim 15, wherein the method is non-therapeutic or is therapeutic; optionally wherein the method comprises applying the composition to a tooth, between teeth and/or gums.

17. The aqueous topical oral composition according to any of claims 1 to 12 for use in the treatment of an indication; optionally wherein the indication is an oral health condition.

100A

100B

d

102

L

L

P

FIG. 1

104

108

$P_1$

110

112

106

$P_0$

FIG. 2

FIG. 3

FIG. 4

FIG. 5

FIG. 6

FIG. 7A

FIG. 7B

FIG. 7C

FIG. 8

FIG. 9

116

122   120

FIG. 10

122

120

FIG. 11

FIG. 12

FIG. 13

FIG. 14

FIG. 15

Europäisches
Patentamt

European
Patent Office

Office européen
des brevets

# EUROPEAN SEARCH REPORT

**Application Number**

EP 19 21 5655

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2013/338088 A1 (ALUR HEMANT H [US] ET AL) 19 December 2013 (2013-12-19) | 1,2,5,8, 10,12, 15-17 | INV. A61P1/02 A61K9/00 A61K9/06 A61K47/10 A61K47/34 A61K47/36 |
| Y | * paragraphs [0008], [0037]; claims 1-13; figures 1-7; examples 5-6 * | 1-3,5, 7-17 | |
| A | | 4,6 | |
| X | WO 02/41837 A2 (RXKINETIX INC [US]) 30 May 2002 (2002-05-30) | 1,2,5,8, 10,12, 15-17 | |
| Y | * example 1; table 1 * | 1-3,5, 7-17 | |
| A | | 4,6 | |
| X | US 2018/110897 A1 (BUSH MAGGIE [US] ET AL) 26 April 2018 (2018-04-26) | 1,2,5,8, 10 | |
| Y | * paragraphs [0056] - [0057] * | 1-3,5, 7-17 | |
| A | | 4,6 | |

TECHNICAL FIELDS
SEARCHED     (IPC)

A61P
A61K

The present search report has been drawn up for all claims

| Place of search | Date of completion of the search | Examiner |
|---|---|---|
| Munich | 24 June 2020 | Estañol, Inma |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

 

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 19 21 5655

This annex lists the patent family members relating to the patent documents cited in the above-mentioned European search report.
The members are as contained in the European Patent Office EDP file on
The European Patent Office is in no way liable for these particulars which are merely given for the purpose of information.

24-06-2020

| Patent document cited in search report | | | Publication date | Patent family member(s) | | | Publication date |
|---|---|---|---|---|---|---|---|
| US 2013338088 | A1 | | 19-12-2013 | CN | 101557802 | A | 14-10-2009 |
| | | | | EP | 2219608 | A1 | 25-08-2010 |
| | | | | US | 2010324110 | A1 | 23-12-2010 |
| | | | | US | 2013338088 | A1 | 19-12-2013 |
| | | | | WO | 2009073658 | A1 | 11-06-2009 |
| WO 0241837 | A2 | | 30-05-2002 | AT | 316786 | T | 15-02-2006 |
| | | | | AU | 3934202 | A | 03-06-2002 |
| | | | | BR | 0115531 | A | 03-02-2004 |
| | | | | DE | 60117043 | T2 | 13-07-2006 |
| | | | | DK | 1343492 | T3 | 06-03-2006 |
| | | | | EP | 1343492 | A2 | 17-09-2003 |
| | | | | EP | 1609468 | A2 | 28-12-2005 |
| | | | | ES | 2254523 | T3 | 16-06-2006 |
| | | | | JP | 4822652 | B2 | 24-11-2011 |
| | | | | JP | 2004513957 | A | 13-05-2004 |
| | | | | MX | PA03004546 | A | 10-09-2003 |
| | | | | US | 2002119104 | A1 | 29-08-2002 |
| | | | | US | 2004141949 | A1 | 22-07-2004 |
| | | | | US | 2007014860 | A1 | 18-01-2007 |
| | | | | US | 2007014861 | A1 | 18-01-2007 |
| | | | | US | 2007071824 | A1 | 29-03-2007 |
| | | | | WO | 0241837 | A2 | 30-05-2002 |
| US 2018110897 | A1 | | 26-04-2018 | CN | 107683140 | A | 09-02-2018 |
| | | | | US | 2018110897 | A1 | 26-04-2018 |
| | | | | WO | 2016164903 | A1 | 13-10-2016 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **HAMLEY, I. W.** The physics of block copolymers. Oxford University Press, 1998, 1-20